# EUROPEAN PATENT APPLICATION

(11) **EP 4 193 992 A1**
(43) Date of publication of application: **14.06.2023**
(21) Application number: 22212873.8
(22) Date of filing: 12.12.2022
(51) Int. Cl.: A61K 31/197, A61K 36/53, A61P 1/00, A61P 3/00, A61P 25/02, A23L 5/00

(54) **NEW COMPOSITION AND USES THEREOF**

(30) Priority: 13.12.2021 IT 202100031112
(71) Applicant: Depofarma S.p.A., 31022 Preganziol (Treviso) (IT)
(72) Inventor: DALLA ZORZA, Paola, Preganziol (Treviso) (IT); DALLA ZORZA, Alessandra, Preganziol (Treviso) (IT)
(74) Representative: Delbarba, Andrea

(57) **Abstract**

The present invention relates to a composition comprising gamma aminobutyric acid (GABA) and an extract of a plant belonging to the species *Melissa officinalis.* Furthermore, the invention relates to the medical use of the composition, in particular, for treating or preventing a disorder associated with perimenopause or menopause in a subject. The invention also relates to a dietary supplement comprising said composition.

## Description

The present invention relates to a composition comprising gamma aminobutyric acid (GABA) and an extract of a plant belonging to the species *Melissa officinalis.*

### STATE OF THE ART

Menopause occurs in a woman when her ovaries stop producing oestrogen and progesterone. In the years preceding menopause, the production of oestrogen and progesterone begins to decrease and menstruation and ovulation are less frequent, eventually ending altogether; at that point a pregnancy can no longer occur naturally. A woman's last menstruation can be identified only later, after the absence of menstruation (amenorrhoea) for at least one year.

Perimenopause is understood as the period extending from several years before to the year following the last menstrual cycle. The number of years of perimenopause preceding the last menstruation varies considerably. During this phase, oestrogen and progestogen levels fluctuate significantly and it is believed that this phenomenon is responsible for the menopause symptoms experienced by many women after age forty.

During perimenopause and menopause, the symptoms may be absent, modest, moderate or severe.

Hot flushes, i.e. sudden sensations of an increase in body temperature, affect 75 to 85% of women and usually appear before the interruption of the menstrual cycle; they last on average for about 7.5 years, but may also last over 10 years. In general, the intensity and frequency of hot flushes decrease as time passes.

The cause of hot flushes is not known, but it involves a readjustment of the hypothalamus, which controls body temperature. Consequently, even small increases in temperature may cause one to perceive this sensation of heat.

Hot flushes that occur during the night are defined as night sweats. Furthermore, depression, irritability, anxiety, nervousness, sleep disorders (including insomnia), loss of concentration, headache and fatigue may also occur. Many women experience these symptoms during perimenopause and deduce that they are caused by menopause.

Night sweats can disrupt sleep, causing tiredness, irritability, loss of concentration and mood changes. In such cases, the symptoms may be associated indirectly with menopause (through night sweats).

The treatment of menopause is concentrated on relieving symptoms such as hot flushes and vaginal dryness. To date, the most effective therapy is a hormone treatment, i.e. an oestrogen and/or progestin treatment. However, hormone therapy results in numerous side effects that are sometimes not tolerated by the patient.

Effective measures that do not provide for hormones include hypnosis performed by a qualified health care worker to help to alleviate the hot flushes, cognitive behaviour therapy, and treatment with antidepressants and/or gabapentin.

Thus, there is a strongly felt need to have a treatment for alleviating the symptoms of perimenopause and menopause which is well tolerated by patients and does not cause side effects.

### SUMMARY OF THE INVENTION

A first aspect of the present invention relates to a composition comprising gamma aminobutyric acid (GABA) and an extract of a plant belonging to the species *Melissa officinalis.*

A second aspect of the present invention relates to the composition as described above for use as a medicament.

A third aspect of the present invention relates to the composition as described above for use in the treatment or prevention of a disorder associated with perimenopause or menopause in a subject.

A fourth aspect of the present invention relates to the composition as described above for use in the treatment and/or prevention of a pathological intestinal condition in an individual.

A fifth aspect of the present invention relates to the composition as described above for use in the treatment or prevention of a pelvic neuropathy and/or symptoms related to a pelvic neuropathy in an individual.

A sixth aspect of the present invention relates to the composition as described above for use in the treatment or prevention of a metabolic syndrome in an individual.

A seventh aspect of the present invention relates to a dietary supplement comprising the composition as described above in an individual.

### BRIEF DESCRIPTION OF THE FIGURES

Figure 1 shows the rating of abdominal pain in subjects at the start (t0) and after 30 days of treatment (t30) with tablets comprising 250 mg of GABA and 50 mg of an extract of *M.officinalis;*
Figure 2 shows the rating of the intensity of abdominal pain in subjects at the start (t0) and after 30 days of treatment (t30) with tablets comprising 250 mg of GABA and 50 mg of an extract of *M.officinalis;* and
Figure 3 shows the quality of life rating with a 34-question test in subjects at the start (t0) and after 30 days of treatment (t30) with tablets comprising 250 mg of GABA and 50 mg of an extract of *M.officinalis.*

### DETAILED DESCRIPTION OF THE INVENTION

A first aspect of the present invention relates to a composition comprising gamma aminobutyric acid (GABA) and an extract of a plant belonging to the species *Melissa officinalis.*

In one embodiment, the ratio of GABA:*M.officinalis* extract by weight is between 8:1 and 3:1. Preferably, the ratio by weight of GABA:extract of a plant belonging to the species *M.officinalis* is 5:1.

In one embodiment, the extract of a plant belonging to the species *M.officinalis* comprises rosmarinic acid in a concentration of between 1 and 4% weight/weight (w/w), preferably between 3 and 4% w/w.

The extract of at least one plant belonging to the species *M.officinalis* is preferably obtained from at least one part of said plant, preferably from at least one leaf of said plant. In one embodiment, the extract is obtained through a process that comprises at least one step of hydroalcoholic extraction from at least one leaf of said plant. The hydroalcoholic extraction is preferably obtained thanks to a solution comprising at least 70% volume/volume (v/v) of alcohol, preferably ethyl alcohol.

In a preferred embodiment, the extract of a plant belonging to the species *M.officinalis* is a dry extract, i.e. it undergoes at least one drying step after extraction. The extract of a plant belonging to the species *M.officinalis* preferably has a particle size of between 200 and 350 microns (µm), more preferably at least 90% of the dry extract has a particle size of between 250 and 320 µm.

In one embodiment, the composition of the present invention comprises at least one excipient accepted for pharmaceutical use and/or which may be used in the preparation of the composition and is biologically safe and nontoxic.

In a further embodiment of the invention, the composition consists in GABA and the extract of a plant belonging to the species *M.officinalis* and at least one excipient accepted for pharmaceutical use. In other words, the composition comprises GABA and the extract of a plant belonging to the species *M. officinalis* as the only active ingredients.

In one embodiment, the composition is formulated for oral administration. Preferably, the composition is formulated as a tablet, preferably an extended-release tablet, capsule, pill, granular powder, hard-shelled capsule, orodispersible granules, sachet, confection or drinkable vial.

In another embodiment, the composition is formulated as a liquid, preferably as a syrup or as a drink or drinkable vials.

A second aspect of the present invention relates to the composition as described in detail above for use as a medicament.

The Applicant has in fact discovered that the composition of the present invention is capable of preventing and/or treating several disorders or pathologies. In particular, the Applicant has surprisingly discovered that the composition of the present invention reduces and alleviates the disorders and symptoms related to or associated with menopause or perimenopause. In other words, the administration of the composition of the present invention significantly reduces disorders such as sudden sensations of increased body temperature, sleep disturbances, mood disturbances, such as feelings of anxiety or excessive irritability. Furthermore, the composition of the present invention has demonstrated to be effective in preventing and/or treating a pathological intestinal condition. In particular, thanks to regular administration of the above-described composition, an individual affected by a pathological intestinal condition is able to reduce symptoms related to said pathology. Finally, the composition of the present invention demonstrates to be a promising alternative to the therapies known to date for treating or preventing a pelvic neuropathy or a metabolic syndrome.

A third aspect of the present invention thus relates to the composition as described in detail above for use in the treatment or prevention of a disorder associated with perimenopause or menopause in a subject. Said disorder associated with perimenopause or menopause is preferably selected from: a sleep disturbance, preferably night awakenings, sudden sensations of increased body temperature and mood disturbance, preferably increased irritability and anxiety.

A fourth aspect of the present invention relates to the composition as described in detail above for use in the treatment and/or prevention of a pathological intestinal condition in an individual.

Said pathological intestinal condition is preferably selected from: irritable bowel syndrome, diarrhoea, intestinal inflammation, ulcerative colitis, gastric atrophy, intestinal diverticula, stenosis, obstruction and diabetic neuropathy.

A fifth aspect of the present invention relates to the composition as described in detail above for use in the treatment or prevention of a pelvic neuropathy and/or symptoms related to a pelvic neuropathy, preferably vulvodynia or chronic pelvic pain.

A sixth aspect of the present invention relates to the composition as described in detail above for use in the treatment or prevention of a metabolic syndrome.

In one embodiment of the invention, for the medical uses described above, the composition is taken at least once a day, preferably at least twice a day, more preferably at least three times a day. In a preferred embodiment, the composition is taken before meals, at least three times a day. In a further embodiment, the composition is taken once in the morning and twice before bedtime. In another embodiment, the composition is taken once in the morning, once before lunch and once before bedtime.

A seventh aspect of the present invention relates to a dietary supplement comprising the composition as described in detail above.

An eighth aspect of the present invention relates to a method for treating or preventing a disorder associated with perimenopause or menopause in a subject. The method comprises at least one step of administering a composition as described in detail above to an individual who has a need for it.

Said disorder associated with perimenopause or menopause is preferably selected from: a sleep disturbance, preferably night awakenings, sudden sensations of increased body temperature and mood disturbances, preferably increased irritability and anxiety.

A ninth aspect of the present invention relates to a method for treating or preventing a pathological intestinal condition in an individual. The method comprises at least one step of administering a composition as described in detail above to an individual who has a need for it.

Said pathological intestinal condition is preferably selected from: irritable bowel syndrome, diarrhoea, intestinal inflammation, ulcerative colitis, gastric atrophy, intestinal diverticula, stenosis, obstruction and diabetic neuropathy.

A tenth aspect of the present invention relates to a method for treating or preventing pelvic neuropathy and/or symptoms related to a pelvic neuropathy, preferably vulvodynia or chronic pelvic pain. The method comprises at least one step of administering a composition as described in detail above to an individual who has a need for it.

An eleventh aspect of the present invention relates to a method for treating or preventing a metabolic syndrome. The method comprises at least one step of administering a composition as described in detail above to an individual who has a need for it.

In one embodiment of the invention, for the above-described medical uses, the composition is administered at least once a day, preferably at least twice a day, more preferably at least three times a day. In a preferred embodiment, the composition is administered before meals, at least three times a day. In a further embodiment, the composition is administered once in the morning and twice before bedtime. In another embodiment, the composition is administered once in the morning, once before lunch and once before bedtime.

### EXAMPLE

### Observational study on 35 women in menopause or perimenopause

### Inclusion criteria:

- Perimenopause or menopause;
- At least 5 sudden sensations of heat per day;
- Sudden sensations of heat of a moderate or intense degree.

Treatment with tablets comprising 250 mg of GABA and 50 mg of extract of *Melissa officinalis* for 30 days:
- 1 tablet in the afternoon; and
- 2 tablets before going to bed.

### Results

Reduction in the sudden sensations of heat -73% in frequency; -60% in intensity and -53% in duration.
Daily wellbeing: - 77% irritability; - 59% anxiety and nervousness and + 72% concentration,
Night-time rest: - 79% insomnia; - 48% night sweats and - 60% night awakenings.

No side effects were reported or detected during the treatment.

### Observational study on patients with irritable bowel syndrome (IBS)

25 patients with IBS (any variant) were evaluated. After their medical history was examined, the patients underwent a determination of their IBSS score and were given a validated 34-question questionnaire on the quality of life. The patients subsequently took tablets comprising 250 mg of GABA and 50 mg of extract of *Melissa officinalis* according to the following regimen: 1 tablet in the morning, one at lunchtime, and one in the evening before going to bed.

They were reassessed after 30 days of having taken the product.

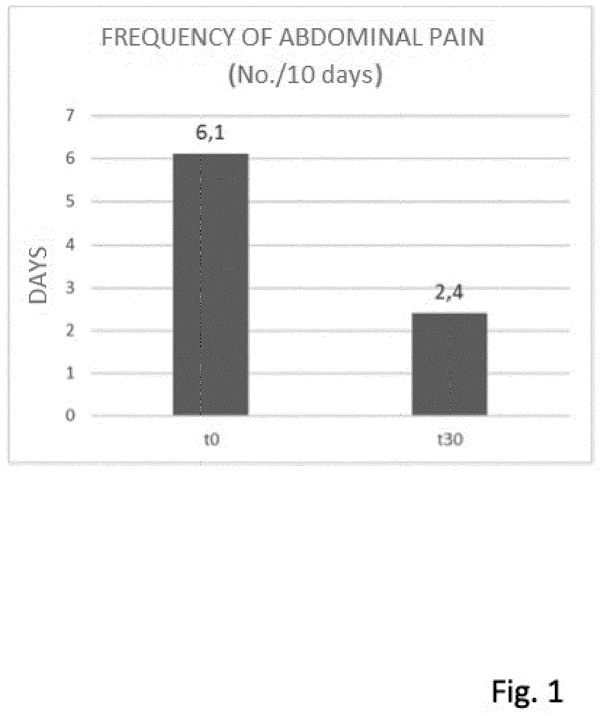

The results show that the frequency of the abdominal pain is drastically reduced after treatment (Figure 1); indeed, there is a 60% reduction compared to the start of treatment.

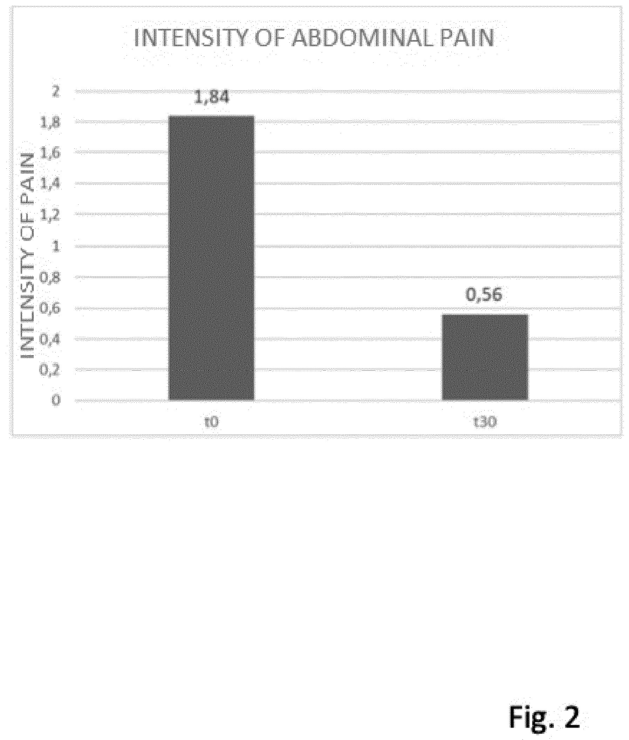

Furthermore, the intensity of abdominal pain is also reduced by about 70% (Figure 2).

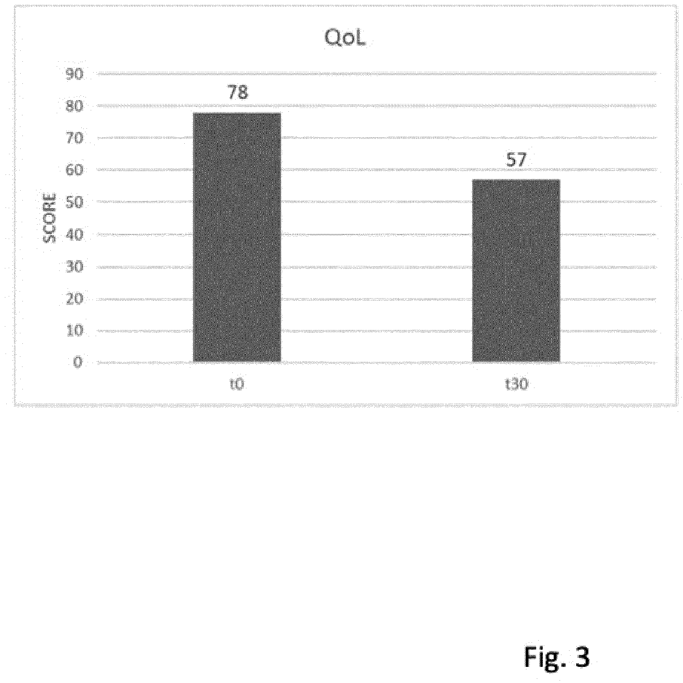

Finally, the quality of life, rated through a 34-question questionnaire, shows a significant increase after treatment (Figure 3).

## Claims

1. A composition comprising gamma aminobutyric acid (GABA) and an extract of a plant belonging to the species *Melissa officinalis,* wherein the ratio of GABA:*M. officinalis* extract by weight is between 8:1 and 3:1.

2. The composition according to claim 1, wherein the ratio by weight of GABA:extract of a plant belonging to the species *M.officinalis* is 5:1.

3. The composition according to claim 1 or 2, wherein the extract of a plant belonging to the species *M.officinalis* comprises rosmarinic acid in a concentration between 1 and 4% w/w (w/w).

4. The composition according to any one of claims 1-3, wherein the extract of a plant belonging to the species *Melissa officinalis* is an extract of at least one leaf of a plant belonging to the species *M.officinalis.*

5. The composition according to any one of claims 1-4, comprising GABA and the extract of a plant belonging to the species *Melissa officinalis* and at least one excipient accepted for pharmaceutical use.

6. The composition according to any one of claims 1-5, comprising at least one excipient accepted for pharmaceutical use.

7. The composition according to any one of claims 1-6, wherein said composition is formulated as a tablet, preferably an extended-release tablet, capsule, pill, granular powder, hard-shelled capsule, orodispersible granules, sachet, confection or drinkable vial, or is formulated as a liquid, preferably as a syrup or as a drink or drinkable vials.

8. The composition according to any one of claims 1-7 for use as a medicament.

9. The composition according to any one of claims 1-7, for use in treating a disorder associated with perimenopause or menopause in a subject.

10. The composition for use according to claim 9, wherein the disorder associated with perimenopause or menopause is selected from: a sleep disturbance, preferably night awakenings, sudden sensations of increased body temperature and mood disturbances, preferably increased irritability and anxiety.

11. The composition according to any one of claims 1-7, for use in the treatment and/or prevention of a pathological intestinal condition selected from: irritable bowel syndrome, diarrhoea, intestinal inflammation, ulcerative colitis, gastric atrophy, intestinal diverticula, stenosis, obstruction and diabetic neuropathy.

12. The composition according to any one of claims 1-7, for use in the treatment or prevention of a pelvic neuropathy and/or symptoms related to a pelvic neuropathy, preferably vulvodynia or chronic pelvic pain.

13. The composition according to any one of claims 1-7, for use in the treatment or prevention of a metabolic syndrome.

14. A dietary supplement comprising the composition according to any one of claims 1-7.
